# DEMANDE DE BREVET EUROPEEN

(11) **EP 4 233 921 A2**
(43) Date de publication de la demande: **30.08.2023**
(21) Numéro de dépôt: 22203415.9
(22) Date de dépôt: 24.10.2022
(51) Int. Cl.: A61L 2/24, G16H 40/20, A61L 2/10, A61L 2/00, G16H 40/63, E06B 11/00

(54) **DISPOSITIF DE DÉCONTAMINATION D'OBJETS ET DE CONTRÔLE DE LA DÉCONTAMINATION**

(30) Priorité: 08.12.2021 FR 2113147
(71) Demandeur: Vinci, Régine, 66000 Perpignan (FR); Llobet, Axel, 66240 Saint Estève (FR)
(72) Inventeur: VINCI, Régine, 66000 Perpignan (FR); LLOBET, Axel, 66240 Saint Estève (FR)
(74) Mandataire: Tzeuton Tchangoum, Eric Olivier

(57) **Abrégé**

L'invention concerne un système de contrôle de décontamination pour un établissement comprenant au moins un local sensible (L), comprenant :
- un décontaminateur (D1) ;
- au moins un badge (I) avec une étiquette NFC, et/ou au moins un objet (T) portant une étiquette NFC ;
- une première horloge (H1) du décontaminateur (D1) ;
- un sabot (S) à l'entrée dudit local (L), comportant un lecteur NFC et une deuxième horloge (H2) synchrone de la première.

Selon l'invention, le système déclenche le décontaminateur (D1) lorsque ledit badge (I) y est apposé et/ou ledit objet (T) y est introduit, et écrit l'horodatage correspondant sur ladite étiquette NFC ; puis autorise une entrée (E) dans ledit local (L) si un premier délai (A) entre l'horodatage et la lecture sur le sabot (S), est inférieur à un deuxième délai (B) seuil ; dans le cas contraire, le système interdit ladite entrée (E).

## Description

La présente description découle de la constatation suivante.

Différents objets, portés par le personnel hospitalier, tels que téléphone portable, montre, porte-clés, bijoux, sont contaminés et suivent ces personnes dans des locaux sensibles, tels que les blocs opératoires.

De nombreuses publications confirment que ces objets sont contaminés par une grande variété de germes et virus pathogènes, avec, pour conséquence, l'émergence de maladies nosocomiales.

Pour lutter contre ce phénomène, le remède serait d'interdire d'introduire ces objets dans les locaux sensibles.

Il semble, toutefois, que certains de ces objets, comme le téléphone sont très utiles, sinon indispensables, dans certaines installations de soins, comme la chirurgie.

Pour lutter contre ces contaminations, des objets vers les patients, le moyen le plus évident consiste à décontaminer ces objets avant qu'ils n'entrent dans les locaux sensibles.

Or, ceci suppose une discipline de la part du personnel, malheureusement, pas souvent respectée.

Ainsi, un objectif de la présente invention est de proposer un système et un procédé de contrôle de décontamination permettant de s'assurer que le personnel hospitalier entre dans des lieux sensibles tels que des blocs opératoires, en ayant décontaminé leurs objets, et de préférence leurs mains.

Pour atteindre cet objectif, l'invention propose un système de contrôle de décontamination pour un établissement comprenant au moins un local sensible, le système de contrôle de décontamination comprenant :
- un décontaminateur ;
- au moins un badge avec une étiquette NFC incluse ou collée sur celui-ci, et/ou au moins un objet portant une étiquette NFC ;
- une première horloge fournissant le jour et l'heure d'une décontamination au moyen du décontaminateur ;
- un sabot à l'entrée dudit local sensible, comportant un lecteur d'étiquettes NFC et étant équipé d'une deuxième horloge synchrone de la première horloge du décontaminateur,
   caractérisé en ce que le système est configuré pour déclencher la décontamination par le décontaminateur lorsque ledit badge y est apposé ou introduit et/ou ledit objet y est introduit, et pour écrire le jour et l'heure de la décontamination sur ladite étiquette NFC par un circuit spécialisé ;
   et en ce que le système est configuré pour autoriser une entrée dans ledit local sensible si un premier délai correspondant au temps passé entre l'heure et le jour de la décontamination, à l'heure et au jour de présentation dudit badge et/ou dudit objet au sabot, est inférieur à un deuxième délai prédéterminé pour considérer une persistance de la décontamination ; dans le cas contraire, le système est configuré pour interdire ladite entrée dans ledit local sensible.

Par « décontaminateur » est entendu un appareillage permettant de décontaminer, stériliser, éliminer substantiellement les germes et autres sources de maladies nosocomiales.

Avantageusement, l'invention permet de conditionner l'accès au local sensible à la décontamination des objets et/ou au lavage des mains, ce qui limite grandement le risque de maladies nosocomiales.

La présente invention repose sur un dispositif innovant, n'autorisant l'entrée de ces objets que s'ils sont décontaminés et/ou les mains sont lavées, et d'une manière stricte et incontournable.

Selon une variante, le décontaminateur forme un distributeur de produit hydroalcoolique, comprenant un récipient d'un produit hydroalcoolique, la décontamination étant le lavage des mains au moyen du produit hydroalcoolique.

Cela permet de s'assurer de la décontamination des mains du personnel de santé.

Selon une variante, le décontaminateur comprend un volume de décontamination refermable par un couvercle. Cela permet de s'assurer de la décontamination des objets transportés par le personnel de santé vers les locaux sensibles. Les objets à décontaminer sont introduits dans l'enceinte pour réaliser une décontamination poussée.

Selon une variante, le système de contrôle de décontamination comprend en outre un récipient d'un produit hydroalcoolique associé au décontaminateur, le système de contrôle de décontamination étant configuré pour asservir l'ouverture du couvercle du décontaminateur et/ou son fonctionnement à une à la distribution automatique et simultanée du produit hydroalcoolique, la décontamination comprenant une exposition des objets à un moyen de décontaminatation dans ledit volume de décontamination, et le lavage des mains au moyen du produit hydroalcoolique.

Cela permet de conditionner l'accès au local sensible à la fois à la décontamination des objets et à l'utilisation du produit hydroalcoolique.

Selon une variante, les horloges sont synchronisées sur un signal radio, de préférence celui de l'émetteur DCF 77 ; ou sur l'heure légale.

Cela permet de s'assurer de la synchronisation parfaite des horloges.

Selon une variante, le sabot comporte un clavier permettant de fixer pour le local sensible correspondant, le délai de persistance supposée de décontamination des objets déposés dans le décontaminateur.

Cela permet de configurer ou reconfigurer aisément le deuxième délai.

Selon une variante, ledit objet comprend un téléphone portable, une montre, un porte-clés, et/ou des bijoux.

Cela permet de limiter les opérations de déplacements et rangements de ces objets, et de limiter les risques de pertes de ces objets. Le téléphone peut être important à décontaminer pour pouvoir l'utiliser dans le local sensible.

Selon une variante, ledit décontaminateur comprend un moyen d'activation pour activer la distribution de produit hydroalcoolique par une détection de main dans une trajectoire de distribution du produit hydroalcoolique.

Cela permet de s'assurer que les mains ont effectivement été en contact avec le produit hydroalcoolique. En outre, cela permet de déclencher la distribution sans contact.

L'invention concerne en outre un établissement comprenant :
- au moins un local sensible, et
- un système de contrôle de décontamination selon l'invention.

Un autre objet de l'invention a trait à un procédé de contrôle de décontamination pour un établissement comprenant au moins un local sensible, au moyen des dispositifs suivants :
- un décontaminateur ;
- au moins un badge avec une étiquette NFC incluse ou collée sur celui-ci, et/ou au moins un objet portant une étiquette NFC ;
- une première horloge fournissant le jour et l'heure d'une décontamination au moyen du décontaminateur ;
- un sabot à l'entrée dudit local sensible, comportant un lecteur d'étiquettes NFC et étant équipé d'une deuxième horloge synchrone de la première horloge du décontaminateur,
caractérisé en ce qu'il comprend :
- une étape de décontamination pour déclencher la décontamination par le décontaminateur lorsque ledit badge y est apposé ou introduit et/ou ledit objet y est introduit, et écrire le jour et l'heure de la décontamination sur ladite étiquette NFC par un circuit spécialisé ;
- une étape d'autorisation pour autoriser une entrée dans ledit local sensible si un premier délai correspondant au temps passé entre l'heure et le jour de la décontamination, à l'heure et au jour de présentation dudit badge et/ou dudit objet au sabot, est inférieur à un deuxième délai prédéterminé pour considérer une persistance de la décontamination ;
- dans le cas contraire, une étape d'interdiction pour interdire ladite entrée dans ledit local sensible.

L'invention sera davantage détaillée sur la base des figures ci-jointes illustrant des modes de réalisations de l'invention, à savoir :

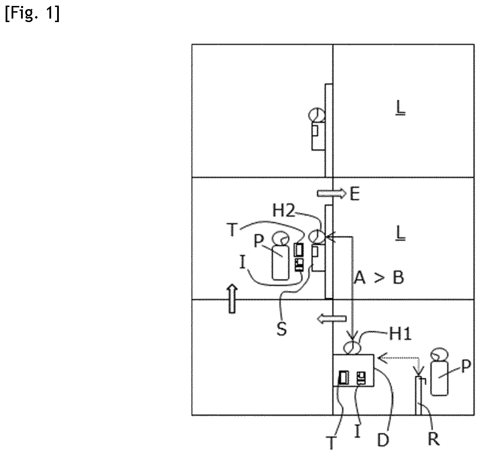

- [Fig. 1] illustre schématiquement un établissement de santé selon un premier mode de réalisation préféré de l'invention avec une décontamination de téléphone et un lavage des mains au gel hydroalcoolique ; et

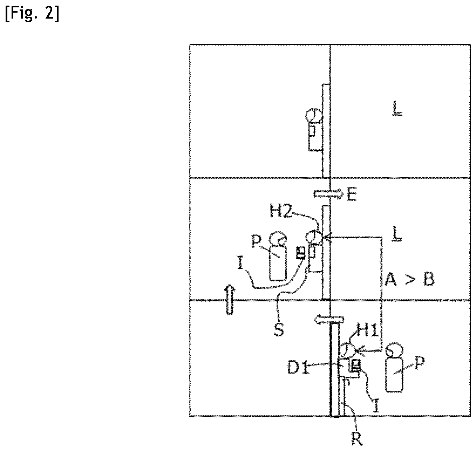

- [Fig. 2] illustre schématiquement un établissement de santé selon un deuxième mode de réalisation avec un lavage des mains au gel hydroalcoolique.

La présente invention repose sur un système innovant, n'autorisant l'entrée (E) de d'objets du personnel de santé P que s'ils sont décontaminés, et d'une manière stricte et incontournable.

Ainsi, le premier mode de réalisation de l'invention propose un système permettant la vérification de la décontamination des mains et autres objets, en vue de maîtriser l'accès des locaux sensibles des établissements de soins. Ce système associe traçabilité des objets T décontaminés, sous forme d'une information véhiculée par l'écoulement du temps compté sur une horloge universelle, et commande l'accès au local sensible L.

Elle repose sur quatre parties physiques :
- un décontaminateur D ;
- un dispositif de traçabilité d'objets T décontaminés utilisant des étiquettes NFC portées par des objets T ;
- une horloge H1 fournissant le jour et l'heure dans le décontaminateur D, cette horloge étant synchrone de toutes les horloges de l'établissement. Ce dernier résultat étant obtenu, par exemple, en se synchronisant sur le signal radio comme l'émetteur DCF 77 ;
- un sabot S à l'entrée du local sensible L, comportant un lecteur d'étiquettes NFC et étant équipé d'une horloge synchrone de l'horloge H1 du décontaminateur D.

Le décontaminateur D est en particulier une enceinte décontaminatrice à UVC (LED) équipée d'un couvercle, d'un verrou de fermeture/ouverture commandé électriquement.

Le décontaminateur D comprend un dispositif électronique d'écriture de l'heure sur le tag NFC.

Le sabot S comprend un moyen pour générer un signal autorisant l'entrée urgente, par exemple un haut-parleur.

A titre d'exemple, non limitatif, de réalisation, le fonctionnement, décrit ci-après, permet de mieux comprendre l'invention.

L'objet à décontaminer choisi dans cet exemple : un téléphone portable T, sur lequel on a collé une étiquette NFC. Le téléphone T ainsi équipé est introduit dans le décontaminateur D.

Simultanément, on déclenche le décontaminateur D et l'écriture du jour et de l'heure sur l'étiquette par un circuit spécialisé.

Après quelques secondes, la décontamination étant terminée, le téléphone T est retiré par la personne qui projette de l'utiliser dans le local sensible L. Cette même personne, pour entrer dans le local sensible L, présente le téléphone T au lecteur du sabot S. Ce dernier, lit l'heure et le jour inscrits sur l'étiquette du téléphone et les compare à l'heure et au jour de son horloge H2, synchrone de l'horloge H1 du décontaminateur D.

Il en déduit le temps passé entre l'heure et le jour d'entrée du téléphone T dans le décontaminateur D à l'heure et au jour de présentation du même téléphone T au sabot S par la personne désirant entrer dans le local sensible L avec ce téléphone T.

Ce délai A, ainsi déterminé, est comparé, par le sabot S, au délai B, décidé pour le local L, délai pendant lequel le téléphone T est considéré comme stérile.

Si A est inférieur à B l'autorisation d'entrer est activée par le sabot S.

Dans le cas contraire, le sabot S affiche un signal d'interdiction d'entrer.

Dans un deuxième exemple de réalisation, non limitatif, l'étiquette NFC est incluse et/ou collée dans la carte badge I portée par le personnel de santé P.

Pour pouvoir entrer dans un local sensible L, le porteur du badge I l'introduit dans le décontaminateur D, comme déjà décrit, et en profite pour y ajouter tous les objets T, qu'il portera dans le local L, contaminés. Par efficacité, avant d'extraire du décontaminateur D le badge I et les objets T, il se désinfectera les mains.

Dans cette configuration, et aussi toutes les autres, on peut asservir l'ouverture du couvercle du décontaminateur D et/ou son fonctionnement, à la distribution automatique et simultanée d'un produit hydroalcoolique de désinfection des mains par un récipient R associé au décontaminateur D, en particulier directement connecté au décontaminateur D.

Dans un troisième exemple de réalisation, non limitatif, l'heure et le jour de l'horloge H1 du décontaminateur D et celle (H2) du sabot S, à l'entrée du local L, sont initialisées à partir de l'heure légale, sont donc synchrones, leur durée de fonctionnement, grâce à une pile de longue durée, dépassant 10 ans, et ne dériveront pas de plus de 1 seconde pour ces 10 ans, grâce aux technologies des montres actuelles et horloges pilotées par quartz.

Le sabot S de lecture, en entrée du local sensible L comporte, en outre, un clavier permettant de fixer pour ce local L, le délai B de persistance supposée de décontamination des objets T déposés dans le décontaminateur D.

Le temps pendant lequel les mains sont réputées décontaminées est programmable pour chaque local sensible L, sur le sabot S comportant le lecteur de l'étiquette.

Le sabot S comporte un circuit délivrant un signal d'autorisation, ou d'interdiction d'entrer dans le local.

En conclusion, ce dispositif, objet de l'invention, est innovant de par son principe utilisant comme identifiant des objets T décontaminés, l'heure et le jour de la décontamination, la traçabilité, grâce au choix de l'identifiant. En outre, il ne nécessite aucune liaison radio ou filaire entre le décontaminateur D et l'entrée du local.

Le système peut comprendre un dispositif informatisé pour analyser la fréquence de la fréquentation du système.

L'information est véhiculée par l'écoulement du temps. Il est universel de par ces choix, utilisable en tous lieux du monde, tel que conçu, avec un minimum de contraintes, tant technologiques qu'humaines pour un prix modeste.

Le deuxième mode de réalisation de l'invention est similaire au premier, en mettant l'accent sur la désinfection des mains, mais la décontamination d'objets n'y est pas prévue.

Ainsi, le système de contrôle d'accès est simplifié (sans le décontaminateur D à UVC). Le décontaminateur D1 utilisé est un distributeur de produit hydroalcoolique.

Le système fonctionne de la manière suivante :
- poser le badge I (que porte chaque membre du personnel P) sur le distributeur de gel D1 muni de l'électronique d'écriture de l'heure ;
- présenter les mains sous le jet de gel, déclenché par une cellule, le signal de déclenchement du gel commandant alors l'écriture de l'heure sur le badge I ;
- le badge I ainsi écrit est utilisé pour le contrôle sur le sabot S, comme déjà décrit.

Les deux dispositifs D, D1 peuvent exister simultanément, suivant les besoins des locaux sensibles L.

Dans la variante préférée, le décontaminateur D1 peut être déclenché soit manuellement, soit par détection des mains sous une trajectoire de distribution du produit hydroalcoolique. Il s'agit par exemple d'un élément d'activation couplé à un capteur, tel qu'un capteur à infra-rouge.

## Revendications

1. Système de contrôle de décontamination pour un établissement comprenant au moins un local sensible (L), le système de contrôle de décontamination comprenant :
- un décontaminateur (D ; D1) ;
- au moins un badge (I) avec une étiquette NFC incluse ou collée sur celui-ci, et/ou au moins un objet (T) portant une étiquette NFC ;
- une première horloge (H1) fournissant le jour et l'heure d'une décontamination au moyen du décontaminateur (D ; D1) ;
- un sabot (S) à l'entrée dudit local sensible (L), comportant un lecteur d'étiquettes NFC et étant équipé d'une deuxième horloge (H2) synchrone de la première horloge (H1) du décontaminateur (D ; D1),
**caractérisé en ce que** le système est configuré pour déclencher la décontamination par le décontaminateur (D ; D1) lorsque ledit badge (I) y est apposé ou introduit et/ou ledit objet (T) y est introduit, et pour écrire le jour et l'heure de la décontamination sur ladite étiquette NFC par un circuit spécialisé ;
et **en ce que** le système est configuré pour autoriser une entrée (E) dans ledit local (L) sensible si un premier délai (A) correspondant au temps passé entre l'heure et le jour de la décontamination, à l'heure et au jour de présentation dudit badge (I) et/ou dudit objet (T) au sabot (S), est inférieur à un deuxième délai (B) prédéterminé pour considérer une persistance de la décontamination ; dans le cas contraire, le système est configuré pour interdire ladite entrée (E) dans ledit local sensible (L).

2. Système de contrôle de décontamination selon la revendication précédente, dans lequel le décontaminateur (D1) forme un distributeur de produit hydroalcoolique, comprenant un récipient (R) d'un produit hydroalcoolique, la décontamination étant le lavage des mains au moyen du produit hydroalcoolique.

3. Système de contrôle de décontamination selon la revendication 1, dans lequel le décontaminateur (D) comprend un volume de décontamination refermable par un couvercle.

4. Système de contrôle de décontamination selon la revendication précédente, comprenant en outre un récipient (R) d'un produit hydroalcoolique associé au décontaminateur (D), le système de contrôle de décontamination étant configuré pour asservir l'ouverture du couvercle du décontaminateur (D) et/ou son fonctionnement à une à la distribution automatique et simultanée du produit hydroalcoolique, la décontamination comprenant une exposition des objets (T) à un moyen de décontaminatation dans ledit volume de décontamination, et le lavage des mains au moyen du produit hydroalcoolique.

5. Système de contrôle de décontamination selon l'une des revendications précédentes, **caractérisé en ce que** les horloges (H1, H2) sont synchronisées sur un signal radio, de préférence celui de l'émetteur DCF 77 ; ou sur l'heure légale.

6. Système de contrôle de décontamination selon l'une des revendications précédentes, dans lequel le sabot (S) comporte un clavier permettant de fixer pour le local sensible (L) correspondant, le délai (B) de persistance supposée de décontamination des objets (T) déposés dans le décontaminateur (D).

7. Système de contrôle de décontamination selon l'une des revendications précédentes, dans lequel ledit objet comprend un téléphone portable (T), une montre, un porte-clés, et/ou des bijoux.

8. Système de contrôle de décontamination selon l'une des revendications précédentes, dans lequel ledit décontaminateur (D1) comprend un moyen d'activation pour activer la distribution de produit hydroalcoolique par une détection de main dans une trajectoire de distribution du produit hydroalcoolique.

9. Etablissement comprenant :
- au moins un local sensible (L), et
- un système de contrôle de décontamination selon l'une des revendications précédentes.

10. Procédé de contrôle de décontamination pour un établissement comprenant au moins un local sensible (L), au moyen des dispositifs suivants :
- un décontaminateur (D ; D1) ;
- au moins un badge (I) avec une étiquette NFC incluse ou collée sur celui-ci, et/ou au moins un objet (T) portant une étiquette NFC ;
- une première horloge (H1) fournissant le jour et l'heure d'une décontamination au moyen du décontaminateur (D ; D1) ;
- un sabot (S) à l'entrée dudit local sensible (L), comportant un lecteur d'étiquettes NFC et étant équipé d'une deuxième horloge (H2) synchrone de la première horloge (H1) du décontaminateur (D ; D1),
**caractérisé en ce qu'**il comprend :
- une étape de décontamination pour déclencher la décontamination par le décontaminateur (D ; D1) lorsque ledit badge (I) y est apposé ou introduit et/ou ledit objet (T) y est introduit, et écrire le jour et l'heure de la décontamination sur ladite étiquette NFC par un circuit spécialisé ;
- une étape d'autorisation pour autoriser une entrée (E) dans ledit local sensible (L) si un premier délai (A) correspondant au temps passé entre l'heure et le jour de la décontamination, à l'heure et au jour de présentation dudit badge (I) et/ou dudit objet (T) au sabot (S), est inférieur à un deuxième délai (B) prédéterminé pour considérer une persistance de la décontamination ;
- dans le cas contraire, une étape d'interdiction pour interdire ladite entrée (E) dans ledit local sensible (L).
